**Europäisches Patentamt**

**European Patent Office**

**Office européen des brevets**

(11) Publication number: **0 247 012**
**A2**

(12) # EUROPEAN PATENT APPLICATION

(21) Application number: **87850162.6**

(22) Date of filing: **14.05.87**

(51) Int. Cl.³: **A 61 F 5/02**

(30) Priority: **15.05.86 SE 8602226**

(43) Date of publication of application:
**25.11.87 Bulletin 87/48**

(84) Designated Contracting States:
**AT BE CH DE ES FR GB GR IT LI LU NL SE**

(71) Applicant: **Andersson, Roland**
**Hantverkargatan 4**
**S-722 12 Vasteras(SE)**

(72) Inventor: **Andersson, Roland**
**Hantverkargatan 4**
**S-722 12 Vasteras(SE)**

(74) Representative: **Lindblom, Erik J.**
**Skördevägen 88**
**S-122 35 Enskede(SE)**

(54) **Bandage.**

(57) The invention relates to the bandage whose length exceeds its width and which comprises a central part (1a) and a respective bandage-securing end part (1b, 1c) located at each end of the central part. One surface of each securing end part is coated with an adhesive layer. The bandage, or tape, is intended for therapeutic remedial purposes with patients suffering from neck and spinal disorders. When in use, one end (1c) of the bandage or tape is affixed to a section of the skin (2) located in the vicinity of the sacrum, and the other end (1d) is affixed to a section of the skin (3) in the vicinity of the lower or intermediate thoracic vertebrae. The length of the tape or bandage measured between the points of attachment when the spine is fully erect and therewith in a correct position is shorter than the distance measured between the skin sections when the spine is forwardly arched (4).

Fig. 1

TITLE OF THE INVENTION:     A BANDAGE.

TECHNICAL FIELD.

The present invention relates to a therapeutic device for
use with patients suffering from neck and/or spinal disorders, and more specifically to a therapeutic device
which has the form of a tape or bandage having
greater length than width

The therapeutic device is of the kind which includes a
central portion and two securing portions, one at each
end of the central portion, and with which solely the
securing portions of the device are provided with an
adhesive layer on one of the surfaces thereof.  Hereinafter the device is referred to as a bandage or tape for
the sake of simplicity.

The inventive bandage is not intended       for use as
a surgical dressing or covering, to protect minor skin
injuries or abrasions, but is intended more particularly
for other purposes,    such   as   for       fixative purposes in the medical treatment of spinal and neck disorders.

BACKGROUND PRIOR ART.

Various types of surgical plasters, dressings, bandages,
etc., are known to the art, in which the length of the
bandage exceeds its width, and which include a central
portion incorporating surgical gauze or a surgical
dressing, and bandage-securing portions located at respective ends of the central portion and coated on one
surface thereof with an adjesive layer by means of which the bandage or the like can be secured to the skin of the patient.

Examples of the prior art in this regard are found in the US Patent Specifications 2,646,040 and 3,233,608.

Elastic bandages also form part of the prior art in the present context.

For example, reference can be made in this regard to an elastic bandage sold under the trademark "Elastoplast" by Beierdorf AG, Hamburg, Federal Republic of Germany. This bandage has a higher degree of elasticity in its longitudinal direction than in its transverse direction, and one surface of the bandage is coated with an adhesive layer.

In the following, the expression "elastic part" is used to define a part of the bandage whose elasticity is equal to or substantially equal to the elasticity of the elastic bandage in its length direction, and the expression "inelastic part" is used to define a part whose elasticity is equal to, or substantially equal to, the elasticity of the bandage in its transverse direction.

The securing tape sold 3M, USA, under the trade name "Durapore" is also considered here to constitute an inelastic material which has an adhesive coating on one side thereof.

The bandage described and illustrated in US Patent Specification 3,194,234 also forms part of the prior art in the present context. This known bandage has a trapezium-shaped central part, which incorporates pockets for accommodating metal stiffening rods, and elastic parts located at respective ends of the central part.

DISCLOSURE OF THE PRESENT INVENTION.

## TECHNICAL PROBLEMS

When considering the prior art described above it becomes apparent that a technical problem resides in the provision of a bandage which can be stretched effectively around a part of the body which is naturally liable to bend, such as an articulated body part, e.g. the knuckles, or the spine, etc., and which bandage comprises a central part and two bandage-securing end parts, and which will remain firmly in position despite the tension forces which prevail at the bandage-securing ends, and which will not require a strong adhesive, liable to cause irritation of the skin, to be applied to the securing ends of the bandage.

Another technical problem in this regard is one of providing bandages of this kind with means effective to dampen and reduce the tension forces generated between the securing ends of the bandage as a result of rapid body movements.

It will also be seen that a further technical problem is one of providing, with simple means, conditions which enable the degree of damping, the degree of reduction, and the course taken by said damping to be pre-selected as a function of the extent to which the bandage is extended.

When a bandage of this kind is to be used as a surgical fixating or supporting bandage, a predominant problem resides in the provision of conditions which, when the bandage is applied to the skin of a person, will ensure that no form of discomfort is experienced when the part of the body to which the bandage is applied is bent or flexed within acceptable limits, and which will present the person concerned with a gentle warning, in the form of a slight feeling of discomfort, when this bending or flexural movement approaches unacceptable limits, and which will cause the person concerned to experience extreme discomfort when these unacceptable limits are exceeded.

With regard to a bandage of this kind, and in particular when the bandage is to be used in a supportive or fixative context, i.e. as a surgical support or fixation, in the treatment of patients having spinal and neck disorders, a pronounced technical problem resides in the provision, with the aid of simple means, of conditions which will enable the adhesive coated securing ends of the bandage, when attached to a patient, to take-up tension forces occurring in said bandage-securing ends, so as to prevent the ends from loosening or being displaced relative to the skin of the person concerned.

Another technical problem is one of providing conditions which will enable such a bandage to be used as a fixation bandage without needing to use a strongly adhesive layer which might irritate the skin tissue, particularly in the case of people with sensitive skin.

Another technical problem in this regard is one of providing, with simple means, conditions which will enable the bandage to comprise a narrow, woven, central part without risk of the bandage snapping as a result of the forces generated in the fixation bandage upon rapid body movements.

A further technical problem is one of providing a fixation bandage which can be readily applied and which is suitably adapted for use by both the therapist and the patient.

Another technical problem resides in the provision of a fixation bandage which will not cause discomfort to the patient upon movement within acceptable limits.

This will avoid the higher muscular activity, detectable by EMG-examination, that is induced when a conventional fixation bandage or tape is used and is experienced after a short while as pronounced tiredness in the spine.

A further technical problem in the present context is one of providing a complete bandage or fixation tape having two bandage-securing ends and a central portion and comprising one or more elastic parts, which may well include the bandage-securing ends, so formed that the elasticity of said parts decreases proportionally and/or non-proportionally in relation to the extent to which the bandage is extended.

A further technical problem is one of providing a bandage or fixation tape which comprises one or more elastic parts or portions thereof which exhibit elastic properties solely during a given degree of bandage extension and thereafter exhibit non-elastic properties.

Still a further technical problem resides in providing a bandage with which the adhesive layers on the bandage-securing end parts thereof can be fastened to the skin without causing irritation and the occurence of so-called tension blisters in this region of the skin, even when the bandage is subjected to high, momentarily acting tension forces.

## SOLUTION

The present invention relates to a bandage having a length which preferably exceeds its width and comprising a central part and bandage-securing end parts each of which is located at a respective end of the central part, each of said bandage-securing end parts having an adhesive layer coated on one surface thereof, for attachment of the bandage to separate areas of the skin.

In accordance with the invention, one bandage-securing end part is intended to be affixed to an area of the skin in the vicinity of the sacrum (vertebrae sacrales); in that the other bandage-securing end is intended to be affixed to part of the skin in the vicinity of the lower or intermediate thoracic vertebrae (vertebrae thoracicae); and in that the length of bandage extending between the bandage-attachment ends when the spine is straight and in a correct posture is slightly shorter than the distance between the aforesaid parts of the skin when the spine is in a forwardly curved posture.

In accordance with one preferred embodiment of the invention, the bandage includes between one bandage-securing end and said central part one or more elastic parts which exhibit elastic properties in both the longitudinal direction and the transverse direction of the bandage. Preferably, there is provided between at least one of the bandage-securing ends and the central part of the bandage a portion which is more elastic than the central part and/or the bandage-securing ends.

The elastic part of the bandage preferably comprises a material, either a natural and/or a treated material, whose elasticity decreases when the material is extended, i.e. in tension. This decrease in elasticity may, e.g., be proportional, or substantially proportional to the extent to which the bandage is extended or lengthened.

It is proposed, particularly when the bandage is to serve as a fixation bandage for treating patients with neck and spinal disorders, that the central part of the bandage is narrower than the bandage-securing ends, and that the elastic part has a tapered portion facing towards the central part.

In accordance with a further advantageous embodiment of the invention, the bandage-securing end parts present a recessed portion remote from the central part of the bandage, therewith to afford improved and more comfortable adhesion when the bandage is subjected to tension forces.

It also lies within the scope of the invention for the central part of the bandage and the elastic parts thereof to be formed integrally with one another during manufacture and to be free from an adhesive layer.

It is particularly proposed that the properties of respective elastic parts of the bandage are such as to ensure that said parts will stretch elastically within a given range of bandage extension when subjected to tension forces and that said parts will become inelastic when subjected to forces which tend to stretch the bandage beyond this range of bandage extension.  The elasticity of said elastic parts is such as to ensure that the bandage is restored to its original length, in use, when the forces are removed.

Preferably, the bandage has a length greater than 15 cm and shorter than 50 cm, preferably between 20 cm and 40 cm and/or the central part of the bandage has a length greater than 10 cm.

As will be understood, the distance between the surfaces of the skin to which the bandage-securing end parts are affixed will increase when the spine is curved forwards, and hence it is suggested that with the spine straight, or substantially straight, this distance corresponds to the distance between the bandage-securing end parts when the elastic parts are fully stretched.

## ADVANTAGES

Those advantages primarily associated with a bandage according to the present invention reside in the possibility of using the bandage as a fixation bandage or supporting bandage for the treatment of patients having neck and spine disorders, the inventive bandage being capable of absorbing high tension forces occurring on the bandage-securing end parts, so as to ensure that the said end parts are not displaced to any appreciable extent relative to the skin of the patient and thereby cause irritation and/or so-called tension blisters.

Another advantage afforded by a bandage according to the invention is that the patient is not impeded in safe positions, and hence the patient does not experience an increase in muscular activity, as would be the case if a conventional bandage of this kind was used.

A further important advantage is that the time taken for rehabilitation is much shorter when using an aid constructed in accordance with the invention.

-------------------------------

The primary characteristic features of a bandage according to the present invention are set forth in the characterizing clause of the following claim 1.

-------------------------------

BRIEF DESCRIPTION OF THE DRAWING

An exemplifying embodiment of a bandage according to the invention and intended for use as a therapeutic fixation bandage will now be described in more detail with reference to the accompanying schematic drawings, in which

Figure 1 is a side view of a patient to which a fixation bandage or tape according to the invention has been applied to a patient suffering from a neck and spinal disorder, the patient being illustrated in an upright posture, with the spinal column straight;

Figure 2 is a side view similar to Figure 1 but with the patient in a stooping posture and the fixation bandage extended accordingly; and

Figure 3 is a plan view of a fixation tape or bandage constructed in accordance with the invention.

DESCRIPTION OF EMBODIMENTS AT PRESENT PREFERRED

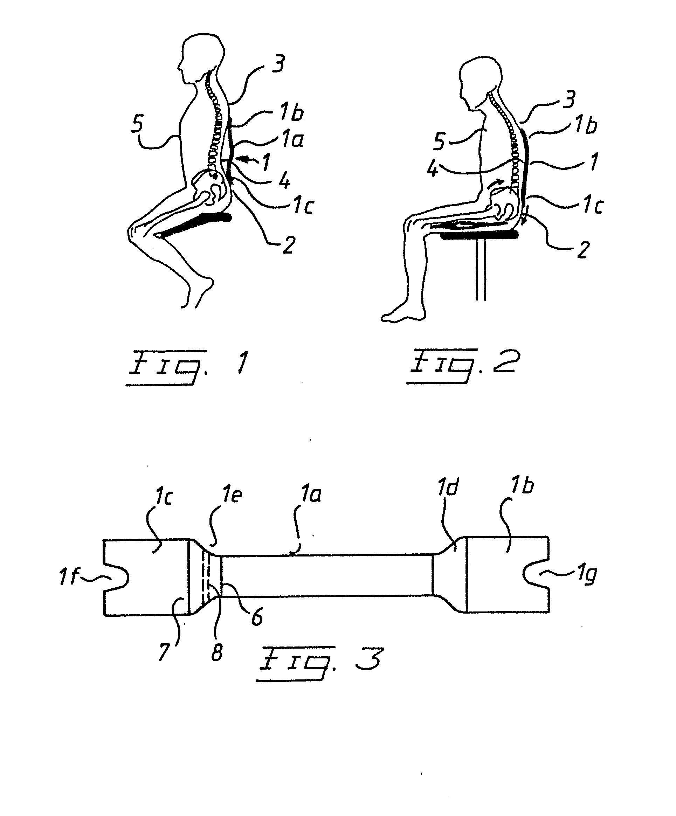

Figure 1 illustrates schematically a patient who is wearing a fixation bandage or tape constructed in accordance with the present invention, for treatment of a neck and spinal disorder, the length of the bandage being greater than its width.  The bandage is referenced 1 and has a central part 1a and two bandage-securing end parts 1b, 1c located at respective ends of the central part.  In this embodiment only the end parts 1b, 1c of the bandage are assumed to be coated with an adhesive layer on one side thereof.

It is assumed that the end part 1c of the bandage is affixed to an area of skin 2 located in the vicinity of the sacrum, while the end part 1b of the bandage is affixed to a skin area 3 in the vicinity of the lower or intermediate thoracic vertebrae.

It will be seen from the Figure that the bandage, or tape, is secured in position so that the length of the bandage between the bandage-securing end parts 1b and 1c in a correct spine position (Figure 1) is somewhat shorter than the shortest distance between the skin areas 2 and 3 as measured with the spine in a forwardly curved or stooping posture as illustrated at 4 in Figures 1 and 2. This is illustrated in Figure 1 by the fact that the bandage 1 is spaced from the small of the back when the patient is in an upright position. Thus, when the patient is in an upright position, with the spine straight, the bandage or tape 1 will not be fully stretched between the skin areas 2 and 3.

Figure 2 illustrates how the fixation bandage 1 is stretched or tensioned when the spine is curved to an undesirable posture in relation to a completely erect posture of the spine.

When the spine of the patient takes a "safe" posture (Figure 1) he or she is unable to feel the presence of the bandage. On the other hand, when the spine of the patient is moved towards a deliterious or harmful position, the bandage or tape will tighten against the back of the patient therewith impeding further stooping of the patient. If the patient ignores this initial warning, the bandage or tape will become extremely tight along the back of the patient as he/she stoops further forward. This feeling of interference as the spine is stooped is more or less proportional to the degree to which the patient stoops, or slouches.

According to the present invention the bandage incorporates between at least one bandage-securing end part and the central part a portion 1d and 1e respectively whose elasticity exceeds the elasticity of the inelastic central part 1a.

The bandage-securing end parts may also have the same elastic properties.

The elastic part 1d or 1e is formed from a material which is elastic in both the longitudinal and the transverse direction.

The elastic part comprises a material, either a natural and/or a treated material, whose elasticity decreases when the material is stretched in its longitudinal direction.

This decrease in elasticity may advantageously be proportional or substantially proportional to the extent to which the material is stretched.

This is achieved with the Figure 3 embodiment in which the central part of the bandage is narrower than the end part thereof and in which the elastic part tapers towards the central part of the bandage or tape.

The bandage-securing end parts 1b, 1c have formed therein a respective recess 1f and 1g, which face away from the central part 1a of the bandage.

Preferably, the elastic part of the inventive bandage has an elasticity which increases gradually in the longitudinal direction of the bandage.

It is suggested that the central part 1a of the bandage is totally inelastic, both in the longitudinal direction and the transverse direction.

The bandage-securing end parts 1b, 1c are coated on one side thereof with an adhesive which will not harm the skin and which will adhere firmly thereto.

It is proposed that no adhesive layer is applied to the central part 1a and the two elastic parts.

It is also proposed that the bandage according to the present invention is produced from a synthetic fabric of the type which allows the skin to breath and which will dry quickly should it become wet,and is resistant to tearing, for example silk fabric.

It is particularly recommended that the end parts 1b, 1c of the bandage are formed to provide the best possible adhesion to the skin and to ensure that the adhesion surface causes the least possible irritation and does not become wrinkled by the skin when the centre part 1a is placed under tension in a manner to cause tension forces to occur between the skin and the attachment part.

The elastic parts dampen considerably the rapidly occurring tension forces        taken-up by the securing parts 1b, 1c. This damping of the tension forces greatly reduces the tendency of the securing parts to be displaced relative to the skin areas 2 and 3.

The following is recommended with regard to the elastic part 1d:

The central part 1a is inelastic and the elasticity of the elastic part 1e may increase gradually from the section 6 to the section 7, therewith forming a fully elastic bandage-securing part 1c.

The central part 1a is inelastic,and is fully elastic, similar to the securing part 1c,within the region from the section 6 to the section 7.

The elastic part 1e may be narrower towards the centre part 1a and wider towards the securing part 1c, in both cases.

Irrespective of which of the embodiments is selected, it is necessary to determine the force required to effect a given extension of the bandage.

It can be assumed that each part section 8 will be elastic over a given length of stretch and then becomes inelastic, so that when the force applied thereto increases those part sections which have become inelastic will increase and "migrate" towards the securing parts 1c.

Restoration takes place completely in the absence of appreciable hysterisis.

It will be understood that the invention is not restricted to the exemplifying embodiments described and illustrated here, and that modifications can be made within the scope of the invention as defined in the following claims.

CLAIMS

1. A bandage whose length is preferably greater than its width and which comprises a central part (1a) and a respective securing end part (1b, 1c) located on each end of the central part and with which bandage solely the bandage-securing end parts are coated on one surface thereof with an adhesive layer for securing the bandage to a section of the skin, characterized in that one securing end part (1b) is intended to be affixed to an arca of the skin (2) in the vicinity of the sacrum; in that the other securing end part (1c) is intended to be affixed to an area of the skin (3) in the vicinity of the lower or intermediate thoracic vertebrae; and in that the length of the bandage or tape (1) between the securing end parts (1b, 1c) when the spine is straight and in a correct erect position (Figure 1) is slightly shorter than the distance between said skin areas when the spine is forwardly curved (4).

2. A bandage according to claim 1, characterized in that one or more elastic parts (1d, 1e) between one securing end part and said central part have elastic properties in both the longitudinal and the transverse direction.

3. A bandage according to claim 1 or 2, characterized in that the elastic part (1b, 1e) comprises a material, a natural and/or a treated material, having an elasticity which decreases as the material is stretched or likewise extended.

4. A bandage according to claim 3, characterized in that the decrease in elasticity of said elastic part is proportional, or substantially proportional, to the extent to which the material is extended.

5. A bandage according to claim 1, 2, 3 or 4, characterized in that the central part (1a) is narrower than the securing end parts (1b, 1c).

6. A bandage according to claim 5, characterized in that the elastic part (1d, 1e) has provided therein a recess which faces towards the central part.

7. A bandage according to claim 1, characterized in that the securing end parts (1b, 1c) have provided therein a recess (1f) which faces away from the central part.

8. A bandage according to any of the preceding claims, characterized in that the central part (1a) and the elastic part (1d, 1e) are formed integrally with one another and have no adhesive layer applied thereon.

9. A bandage according to any of the preceding claims, characterized in that the elastic part (1d, 1e) is arranged to pass into an inelastic state subsequent to being stretched through a pre-determined distance by a load applied thereto, and to return to its original length when the load is removed.

10. A bandage according to claim 1, characterized in that it has a length greater than 15 cm and smaller than 50 cm, preferably between 20 cm and 40 cm and/or that the central part has a length greater than 10 cm.

11. A bandage according to claim 1 or 10, characterized in that the distance between securing end parts is such that the elastic parts are stretched to their limit when the spine is arched forwardly beyond a pre-determined acceptable limit.

Fig. 1

Fig. 2

Fig. 3